# EUROPEAN PATENT APPLICATION

(11) **EP 1 598 765 A1**
(43) Date of publication of application: **23.11.2005**
(21) Application number: 04714482.9
(22) Date of filing: 25.02.2004
(51) Int. Cl.: G06F 19/00

(54) **INFORMATION PROCESSING SYSTEM USING BASE SEQUENCE RELEVANT INFORMATION**

(30) Priority: 27.02.2003 JP 2003051896
(71) Applicant: Hitachi, Ltd., Tokyo 100-8280 (JP)
(72) Inventor: KATO, Takamasa, c/o Hitachi, Ltd., IPG., Chiyoda-ku, Tokyo 100-8220 (JP); MORIMOTO, Takeo, c/o Hitachi, Ltd., IPG., Chiyoda-ku, Tokyo 100-8220 (JP); MATSUO, Hitoshi, c/o Hitachi, Ltd., IPG., Chiyoda-ku, Tokyo 100-8220 (JP); BAN, Hideyuki, c/o Hitachi, Ltd., IPG., Chiyoda-ku, Tokyo 100-8220 (JP); HISAMITSU, Toru, c/o Hitachi, Ltd., IPG., Chiyoda-ku Tokyo 100-8220 (JP); KAMIYAMA, Takuya, c/o Hitachi, Ltd., IPG., Chiyoda-ku, Tokyo 100-8220 (JP)
(74) Representative: Denison, Christopher Marcus
(86) International application number: PCT/JP2004/002212
(87) International publication number: WO 2004/077334

(57) **Abstract**

This invention provides a method for processing information that allows the discovery of a correlation between predetermined individual-related information and nucleotide sequence-related information concerning an individual. This method comprises: step (a) of calculating a percentage for each piece of nucleotide sequence-related information using a first occurrence frequency and a second occurrence frequency, wherein the first occurrence frequency is calculated for each possible piece of nucleotide sequence-related information in a given position in a nucleotide sequence based on a predetermined population and the second occurrence frequency is calculated for each possible piece of nucleotide sequence-related information in the aforementioned position based on the population gathered for predetermined individual-related information concerning an individual; and step (b) of associating the percentage calculated in step (a) with positional information representing the aforementioned position and with the nucleotide sequence-related information for each the predetermined piece of individual-related information.

## Description

### Field of the Invention

The present invention relates to an information processing system that provides information through a communication network.

### Background Technique

Currently, genomic nucleotide sequences of various organisms including humans are being rapidly determined and information on genomic nucleotide sequences is being accumulated in various databases. For example, currently in progress is the construction of a system which will enable various research institutes and researchers to utilize information on genomic nucleotide sequences accumulated in databases through an information network such as the Internet.

At the same time, research for the purpose of genomic drug discovery and analysis of genetic information and the like have been actively conducted using nucleotide sequences contained in such information on genomic nucleotide sequences, and differences in nucleotide sequences among individual organisms represented by the single nucleotide polymorphism are attracting attention. In general, differences in nucleotide sequences among individual organisms refer to a polymorphism defined by existence of a predetermined nucleotide difference at a frequency of 1% or more in an individual species and a variation defined by a predetermined nucleotide difference of less than 1% in an individual species. In particular, known polymorphisms are SNP (single nucleotide polymorphism), in which there is one nucleotide difference among individual organisms; an insertion/deletion polymorphism, in which one to several tens of nucleotides (sometimes several thousands of nucleotides) have been deleted or inserted; VNTR (variable number of tandem repeat), in which the number of repetitions of a sequence comprising two to several tens of nucleotides as one unit varies; and a microsatellite polymorphism (a repetition sequence having about two to four nucleotides).

Such polymorphisms sometimes affect, for example, differences in amino acid sequences of proteins among individual organisms or differences in expression efficiency concerning predetermined genes among individual organisms. Such influences cause, for example, differences in the morbidity rate of predetermined diseases among individual organisms or differences in sensitiveness to predetermined medicaments among individual organisms.

A system, however, which provides semantic information useful for each organism among a plurality of individual organisms through effective utilization of differences in nucleotide sequence-related information, such as a polymorphism, is not yet constructed.

### Disclosure of the Invention

Under the above circumstances, it is an object the present invention to provide a method for processing information on nucleotide sequence that allows discovery of the correlation between predetermined individual-related information and nucleotide sequence-related information concerning an individual.

The method for processing information on nucleotide sequence according to the present invention, whereby the above object has been accomplished, comprises:
step (a) of calculating a percentage for each piece of nucleotide sequence-related information using a first occurrence frequency and a second occurrence frequency, wherein the first occurrence frequency is calculated for each possible piece of nucleotide sequence-related information in a given position in a nucleotide sequence based on a predetermined population and the second occurrence frequency is calculated for each possible piece of nucleotide sequence-related information in the aforementioned position based on the population gathered for predetermined individual-related information concerning an individual; and
step (b) of associating the percentage calculated in step (a) with positional information representing the aforementioned position and with the nucleotide sequence-related information for each predetermined piece of individual-related information.

This method for processing information allows evaluation of the correlation between the individual-related information and the nucleotide sequence-related information by subjecting the percentage calculated in step (a) to relativization based on a predetermined standard value to obtain a relative value. As an absolute value of a difference between the relative value and a predetermined standard value becomes larger, the individual-related information can be determined to have a stronger correlation with the nucleotide sequence-related information.

This method for processing information also allows estimation of relevance among a plurality of pieces of predetermined individual-related information based on a tendency regarding the positional information and the relative value of each piece of nucleotide sequence-related information concerning the individual-related information that had been determined to be correlated with each other.

Also, this method for processing information allows identification of another piece of individual-related information that had been determined to have a tendency of similar for a predetermined individual-related information, which had been determined to have no correlation with nucleotide sequence-related information, and that had been determined to have correlation with nucleotide sequence-related information, and calculation of an indicator for an indirect correlation between the predetermined individual-related information and nucleotide sequence-related information using a value indicating such tendency of similarity and a relative value concerning nucleotide sequence-related information that is correlated with the identified individual-related information.

The method for processing information on nucleotide sequence according to the present invention can be executed in the form of a program that allows a computer comprising hardware, such as a control unit, a transmitter/receiver, and a memory unit, to execute each step of information processing. The method for processing information on nucleotide sequence according to the present invention can be also executed in the form of a recording medium comprising a program that allows a computer comprising hardware, such as a control unit, a transmitter/receiver, and a memory unit, to execute each step of information processing. Further, the method for processing information on nucleotide sequence according to the present invention can be executed in the form of an information processor comprising hardware, such as a control unit, a transmitter/receiver, and a memory unit, that executes each step of information processing.

In addition, the present invention also includes constitutions as disclosed in each claim.

This description includes part or all of the contents as disclosed in the description and/or drawings of Japanese Patent Application No. 2003-051896, which is a priority document of the present application.

### Brief Description of the Drawings

Fig. I schematically shows a construction of a system for providing information to which the present invention has been applied.
Fig. 2 schematically shows a construction of a shared computer.
Fig. 3 shows an embodiment of data that is recorded in a main database ("database" is hereinafter abbreviated to "DB").
Fig. 4 schematically shows a construction of a computer for statistical analysis.
Fig. 5 shows an embodiment of data recorded in a genome-related information DB.
Fig. 6 shows an embodiment of data recorded in an individual-related information DB.
Fig. 7 schematically shows the construction of a personal computer.
Fig. 8 shows an embodiment of the data recorded on the genome-related information recording medium.
Fig. 9 is a flow chart showing a process carried out in a computer for statistical analysis and that in a personal computer in the system for associating individual-related information with genome-related information.
Fig. 10 shows a screen image as an embodiment of an individual-related information collection screen.
Fig. 11 is a flow chart showing a statistical process carried out in a computer for statistical analysis.
Fig. 12 shows an embodiment of data indicating an occurrence frequency of each polymorphism pattern in a polymorphism address calculated from all data stored in genome-related information DB.
Fig. 13 shows an embodiment of data indicating an occurrence frequency of each polymorphism pattern in each polymorphism address concerning individual-related information calculated with the utilization of genome-related information DB and individual-related information DB.
Fig. 14 shows an embodiment of data indicating a percentage of each polymorphism pattern for each polymorphism address concerning individual-related information calculated from the data shown in Fig. 12 and Fig. 13.
Fig. 15 shows an embodiment of data indicating a relative value of each polymorphism pattern for each polymorphism address concerning individual-related information calculated from the data shown in Fig. 14.
Fig. 16 is a flow chart showing processing in a shared computer and a personal computer in a system for providing a morbidity rate of a predetermined disease.
Fig. 17 is a flow chart, which is a continuation of Fig. 16, showing processing in a shared computer and a personal computer in a system for providing a morbidity rate of a predetermined disease.
Fig. 18 is a flow chart showing other processing in a shared computer and a personal computer in a system for providing a morbidity rate of a predetermined disease.
Fig. 19 is a flow chart showing further processing in a shared computer and a personal computer in a system for providing a morbidity rate of a predetermined disease.

### Description of Reference Numerals

1: Communication network
2: Shared computer
3: Personal computer
ST: Computer for statistical analysis

### Preferred Embodiments of the Invention

Hereafter, the present invention is described in detail with reference to the drawings.

In a system for processing information, which provides the morbidity rate of predetermined diseases to a user, a system for creating semantic information is described as an embodiment to which the present invention has been applied. Specifically, the system for processing information creates semantic information, such as morbidity rate, for a user to be provided with based on information concerning an individual (hereafter referred to as "individual-related information").

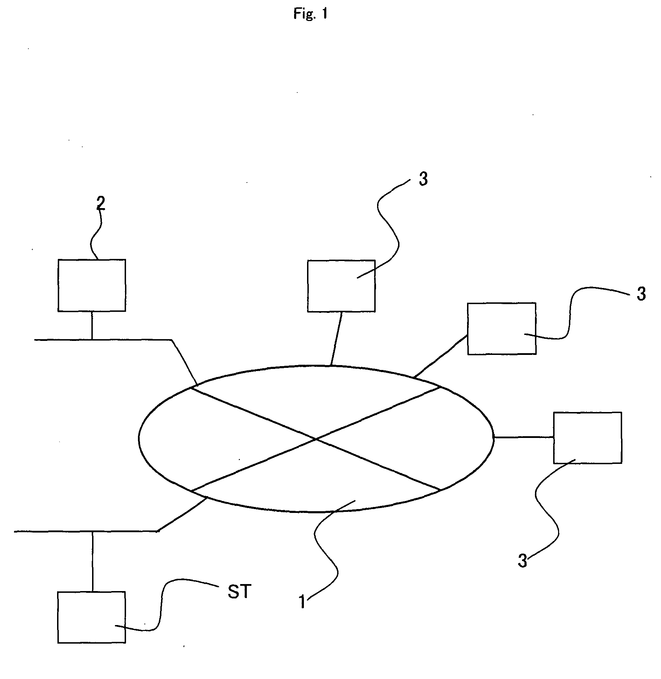

The present embodiment is directed to explaining a system for processing information that creates semantic information based on individual-related information. For the convenience of explanation, this system is explained as a simple model. As shown in Fig. 1, the system for processing information comprises a communication network 1, such as the Internet, a shared computer 2 connected to communication network 1, at least one personal computers 3 connected to communication network 1 and a computer ST for statistical analysis connected to communication network 1, and enables data communication among shared computer 2, personal computers 3 and computer ST for statistical analysis through communication network 1.

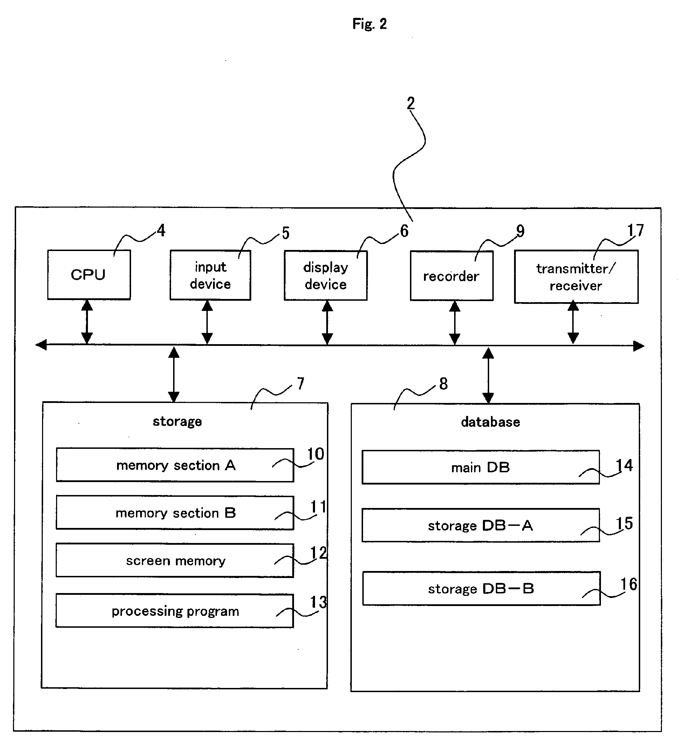

As shown in Fig. 2, shared computer 2 is constituted by a CPU 4 that totally controls the operation of the shared computer 2; an input device 5, such as a keyboard and a mouse, with which information, instructions for executing a program and the like can be input; a display device 6 such as a display apparatus; a storage 7 in which temporary information, unrewritable information and the like are recorded; a database 8 for storing various data; a recorder 9 for writing predetermined information in storage 7 and database 8; and a transmitter/receiver 17 for transmission and reception of information to and from personal computers 3 through communication network 1.

Storage 7 in shared computer 2 is constituted by a memory section A10 and a memory section B11 which respectively record different types of information; a screen memory 12 having recorded therein screen data displayed, for example, on personal computer 3 or display device 6; and a processing program 13 for operating the system. Shared computer 2 may have screen memory 12, processing program 13 and the like in an external recording apparatus (not shown) connected to shared computer 2 through communication network 1 instead of containing those in storage 7 inside shared computer 2.

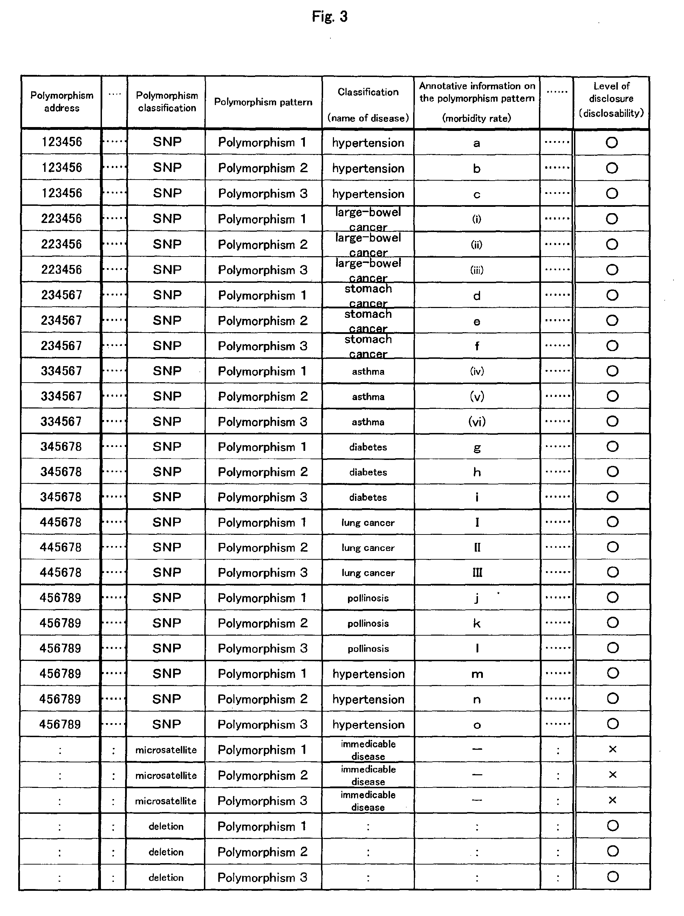

Database 8 (memory) in shared computer 2 is constituted by a main DB 14 in which a polymorphism address, a polymorphism pattern, and semantic information are recorded; a storage DB-A15 for saving information recorded in memory section A10; and a storage DB-B16 for saving information recorded in memory section B11. As shown in Fig. 3, polymorphism addresses, a plurality of possible polymorphism patterns in the polymorphism address respectively, and semantic information implied by each of the plurality of polymorphism patterns respectively are stored in association with one another in main DB 14. Main DB 14 may also have recorded therein semantic information implied by a combination of polymorphism patterns in a plurality of polymorphism addresses (such as haplotype).

The "polymorphism address (positional information)" refers to, at least, a position in a nucleotide sequence where a polymorphism is present. In general, the term "polymorphism" includes, for example, a so-called SNP (single nucleotide polymorphism), RFLP (restriction fragment length of polymorphism), VNTR (variable number of tandem repeat), and microsatellite. However, the term "polymorphism" used herein is not limited to these and also includes a variation in nucleotides and nucleotide sequences existing only at a frequency of less than 1% in an individual species. Therefore, "polymorphism address" also includes a position in a nucleotide sequence which indicates a variation of a nucleotide and nucleotide sequences existing only at a frequency of less than 1% in an individual species. Specifically, the "polymorphism address" indicates a position representing a polymorphism or the like by a combination of numerical values, letters, symbols, and the like. The polymorphism address is not particularly limited, for example, may be represented by a combination of a chromosome number, a symbol indicating a gene having a polymorphism therein, and a numerical value indicating a position of a polymorphism in the gene. Alternatively, it may be a combination of a symbol indicating a gene having polymorphism therein and a numerical value indicating a position of polymorphism in the gene.

Further, a "polymorphism address" may be a notation peculiar to a polymorphism imparted to each polymorphism. When the notation peculiar to a polymorphism is used as a polymorphism address, the polymorphism address does not directly indicate the position in the nucleotide sequence, instead, the position can be indirectly found by the notation peculiar to the polymorphism. Therefore, the "polymorphism address" includes the notation peculiar to the polymorphism.

A "polymorphism pattern (nucleotide sequence-related information)" is information on nucleotide sequences which differ among individual organisms, and contains, at least, a pattern of nucleotides or nucleotide sequences in a polymorphism. In addition, the "polymorphism pattern" includes a pattern of nucleotide and nucleotide sequences existing only at a frequency of less than 1% in an individual species and is not limited to a polymorphism.

For example, in a polymorphism address known to have A or G, the "polymorphism pattern" is represented either by "A" or "G". The "polymorphism pattern" may represent a heterozygote or homozygote in a homologous chromosome. For example, the "polymorphism pattern" can be represented by "AA", "GG", or "AG" in the polymorphism address known to have A or G. Further, the "polymorphism pattern" may indirectly represent a possible pattern in the predetermined polymorphism address instead of direct representation of patterns. For example, in the polymorphism address known to have A or G the "polymorphism pattern" may be represented by "allele 1" when the polymorphism address has "A" or "allele 2" when the polymorphism address has "G". As described above, when the "polymorphism pattern" can be expressed as "AA", "GG", or "AG", the "polymorphism pattern" may be represented by "α" when expressed as "AA", it may be represented by "β" when expressed as "GG", and it may be represented by "γ" when expressed as "AG". When the polymorphism is the microsatellite type the "polymorphism pattern" may be represented, for example, by numerical values indicating "the number of repetitions" and when the polymorphism is the insertion/deletion type the "polymorphism pattern" may be represented, for example, by symbols indicating "presence/absence".

The "polymorphism pattern" in each polymorphism address may be represented by, for example, "polymorphism 1," "polymorphism 2," or "polymorphism 3," in accordance with given rules and arrangements. For example, it can be represented by "polymorphism 1," "polymorphism 2," or "polymorphism 3," in descending order of frequency regarding the "polymorphism pattern" that can appear in each polymorphism address. In this case, "polymorphism 1" in a polymorphism address is not always the same as that in other polymorphism addresses. Specifically, "polymorphism 1" in a given polymorphism address represents "AA" that can appear with the highest frequency, and "polymorphism 1" in other polymorphism addresses represents "GG" that can appear with the highest frequency. In the present embodiment, a "polymorphism pattern" is represented by any of "polymorphism 1," "polymorphism 2," "polymorphism 3," or the like. In the present system, a polymorphism pattern may or may not be encrypted.

The term "semantic information" used herein refers to information associated with the "polymorphism pattern," for example, information including responsiveness to medicaments, side-effect caused by medicaments, a risk against diseases and disorders, diatheses and properties, interaction among proteins, and various phenotypes caused by differences in polymorphism patterns.

"Semantic information" may directly represent a variety of phenotypes resulting from differences in "polymorphism patterns." Alternatively, it may indirectly represent phenotypes with the use of symbols that indicate such phenotypes or the like. "Semantic information" is a type of information which is corrected and increases in the numbers of types accompanied by progress in research on genome and genetics, and constant updating is preferred. In other words, "semantic information" becomes more accurate through increases and decreases in the amount of information accumulated by updating a database using the results of research on genome and genetics.

Information that is further induced from "semantic information" is "information associated with the semantic information" although it is not directly associated with the "polymorphism pattern." When "semantic information" is a risk against diseases, when the relevant risk exceeds a given standard, for example, specific "medical examination items" are derived. These specific "medical examination items" are "information associated with the semantic information."

In the present embodiment, semantic information is recorded in main DB 14 as "annotative information on the polymorphism pattern" associated with at least the predetermined "polymorphism address" and "polymorphism pattern" as shown in Fig. 3. Also, semantic information is associated with, for example, "polymorphism classification," "classification (name of disease)" and the like corresponding to the predetermined "polymorphism address." Consequently, when a predetermined "polymorphism address" is a predetermined "polymorphism pattern," types of diseases and annotative information (semantic information) on the morbidity rates of diseases can be obtained. For example, semantic information can be associated with a combination of respective polymorphism patterns corresponding to a plurality of polymorphism addresses (such as haplotype). In other words, each combination of polymorphism patterns in a plurality of polymorphism addresses can be respectively associated with annotative information (semantic information) representing different morbidity rates for predetermined diseases. In this case, when a plurality of polymorphism addresses are a combination of predetermined polymorphism patterns, annotative information (semantic information) indicating the morbidity rate of a predetermined disease can be obtained.

Semantic information can be further associated with a "level of disclosure" which is set in accordance with a predetermined standard. For example, a standard in setting a "level of disclosure" can be determined by taking into consideration unpredictable disbenefits and the like for individuals that would be caused by disclosure of semantic information, i.e., the morbidity rate of "classification (name of disease)". In particular, in shared computer 2, a "level of disclosure" can be set such that semantic information, the disclosure of which is inappropriate from the view point of, for example, law, regulations, the behavioral norms of an organization having the shared computer 2 or a contract with the user, is not disclosed. In this case, with this system, annotative information representing a morbidity rate associated with a "level of disclosure" at which disclosure is not possible is not disclosed to users. This can prevent the provision of semantic information which could result in unpredictable disbenefit for users or the disclosure of semantic information to parties other than the contract party.

The system may disclose semantic information having a predetermined "level of disclosure" associated therewith to the user through approval by the user of disclosure of semantic information having a predetermined "level of disclosure" associated therewith through, for example, informed consent.

The "level of disclosure" can be set as a plurality of levels of three or more, for example, "1, 2, 3 ... " or "a, b, c ... ". In this case, the level can be set on the shared computer 2 side according to the type of user, such as the user's age, the user's qualification, and whether or not a contract exists with the user. The user can select the level of disclosure such that only annotative information is provided which represents the morbidity rate associated with the level of disclosure that is above (or below) the predetermined level of disclosure determined in accordance with the informed consent or the like.

In database 8, for example, data such as nucleotide sequence-related information that is the genetic information of the individual requester utilizing the system can be recorded in storage DB-B16. In storage DB-A15, for example, data such as information distinguishing the requester from others utilizing the system can be recorded. In this way, the separate recording of the genetic information of individuals and the information for specifying individuals in storage DB-A15 and storage DB-B16, respectively, makes it difficult to associate a user's genetic information with data that specifies the user.

Shared computer 2 is not limited to one having database 8 therein, and it may have an external database (not shown) connected to shared computer 2 through communication network 1. Shared computer 2 may have a plurality of databases 8 therein or may have an internal database 8 and an external database connected to shared computer 2 through communication network 1.

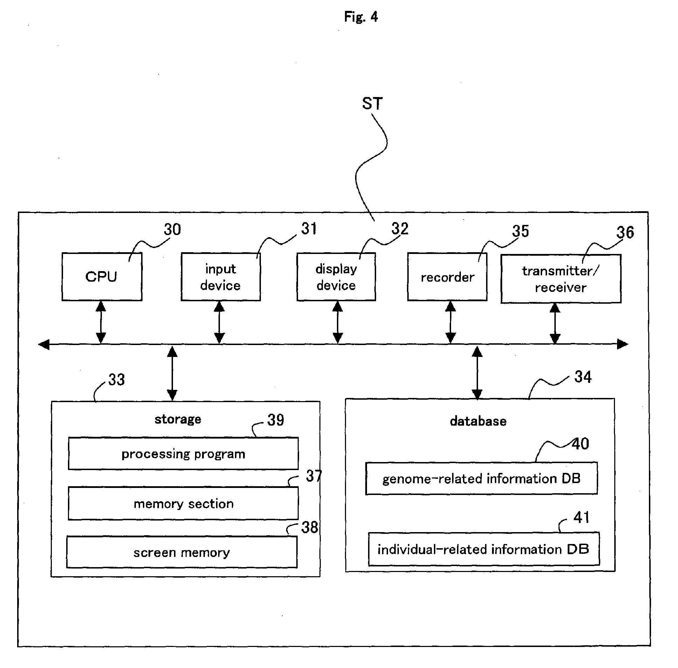

As shown in Fig. 4, a computer ST for statistical analysis is constituted by CPU 30 that totally controls operation of the computer ST for statistical analysis, input device 31, such as a keyboard and a mouse, with which information, instructions for executing a program and the like can be input; display device 32 such as a display apparatus; storage 33 in which temporary information, unrewritable information and the like are recorded; database 34 for storing various data; a recorder 35 for writing predetermined information in storage 33 and database 34; and transmitter/receiver 36 for transmission and reception of information to and from shared computer 2 and personal computers 3 through communication network 1.

Storage 33 in a computer ST for statistical analysis contains recorded therein: memory section 37 for temporarily recording polymorphism patterns and the like transmitted from personal computer 3, results of statistical analysis, or the like; screen memory 38 having, for example, screen data to be displayed on personal computer 3, shared computer 2, or display device 32 recorded therein; and processing program 39 for creating semantic information such as annotative information indicating the morbidity rate of a given disease by having the system operate and using "individual-related information" concerning a plurality of individuals (individual organisms) and "genome-related information" concerning a plurality of individuals (individual organisms). According to this processing program 39, "genome-related information" concerning a plurality of individuals (individual organisms) and "individual-related information" concerning a plurality of individuals (individual organisms) are first accumulated and then, the "genome-related information" and the "individual-related information" are statistically processed. Thus, annotative information (semantic information) or the like indicating the morbidity rate of a given disease can be attained. A computer ST for statistical analysis may comprise processing program 39 or memory section 37 in an external memory (not shown) connected thereto through communication network 1 instead of storage 33 located in the inside thereof.

The term "individual-related information" includes every type of information concerning a predetermined individual, such as properties, psychological conditions, diathesis, physical conditions, health conditions, medical history, lifestyle, patterns of behaving and thinking, habits, and preferences of the individual. Examples of individual-related information include information that is obtained when an individual (an individual organism), such as a user, replies to the previously prepared questions and information that is obtained when a user receives checkups at a medical, testing, or other institution.

The term "genome-related information" refers to a group (a nucleotide sequence-related information group) of data provided by associating a plurality of "polymorphism patterns" concerning a predetermined individual with a predetermined "polymorphism address".

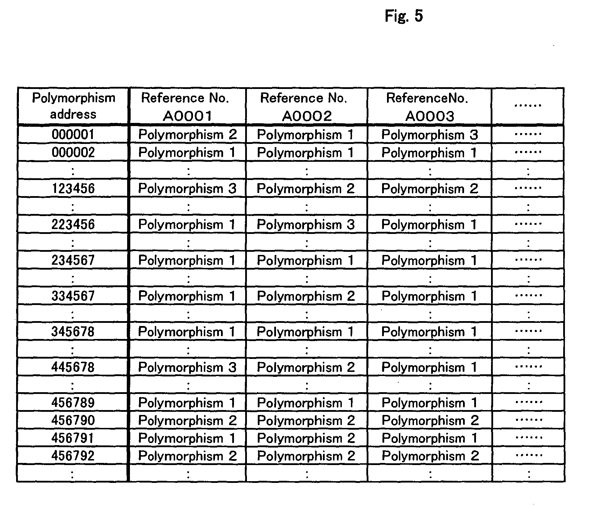

The database 34 (memory) in the computer ST for statistical analysis comprises "genome-related information DB 40" (shown in Fig. 5) having "genome-related information" concerning a plurality of individuals (individual organisms) recorded therein with respect to each individual. The "genome-related information" recorded in the "genome-related information DB 40" is preferably kept anonymous in order to prevent linking to information that directly identifies an individual (individual organism).

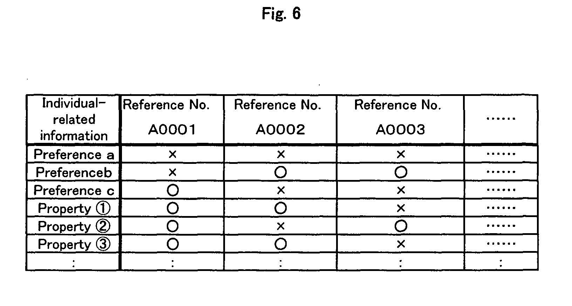

The database 34 comprises "individual-related information DB 41" (shown in Fig. 6) having "individual-related information" received from personal computer 3 recorded therein with respect to each individual (individual organism). The "individual-related information" recorded in the "individual-related information DB 41" is preferably kept anonymous in order to prevent linking to information that directly identifies an individual (individual organism).

The computer ST for statistical analysis is not limited to one having database 34 therein. It may access an external database (not shown) connected thereto through communication network 1.

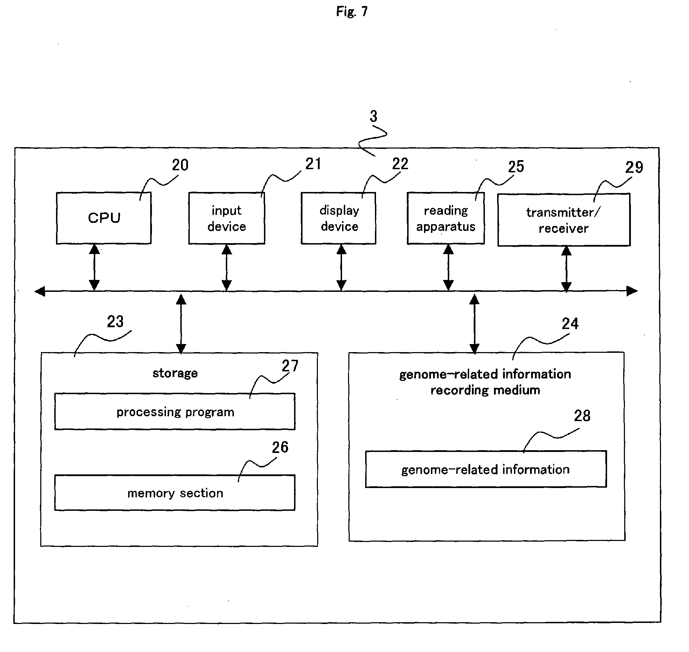

As shown in Fig. 7, personal computer 3 is constituted by CPU 20 that totally controls operation of personal computer 3, input device 21 such as a keyboard and a mouse with which information and instructions for executing a program are input, display device 22 such as a display apparatus, storage 23 having temporary information, rewritable information and the like recorded therein, reading apparatus 25 for reading data from genome-related information recording medium 24, and transmitter/receiver 29 for transmitting and receiving information to and from shared computer 2 through communication network 1. Personal computer 3 is not limited to a commonly used computer. For example, it may be any form of cellular phone, personal digital assistance, or other mobile communication tool.

Storage 23 in personal computer 3 has a memory section 26 for recording information provided from genome-related information recording medium 24 and the like, and is recorded a processing program 27 for operating the system for processing information.

Genome-related information recording medium 24 has genome-related information 28 of an individual recorded thereon. Genome-related information recording medium 24 includes, for example, a magnetic recording medium such as a magnetic disk or a magnetic card, an optical recording medium employing such as a magneto-optic recording system or a phase-change recording system, and a semiconductor memory. This genome-related information recording medium 24 may be in any form such as, for example, card, disk, stick, tape, or drum. Further, this genome-related information recording medium 24 may comprise genome-related information 28 of a single individual (an individual organism) recorded thereon. Alternatively, it may comprise a plurality of pieces of genome-related information 28 on a plurality of individuals (individual organisms) recorded thereon.

Genome-related information 28 contained in genome-related information recording medium 24 refers to, at least, a "polymorphism address" and a "polymorphism pattern" in the predetermined polymorphism address obtained as a result of analysis of an individual's (individual organism's) nucleotide sequences. Genome-related information 28 may contain various information, such as information concerning anamnesis, characteristics, an individual's clinical record, or a result of medical examination.

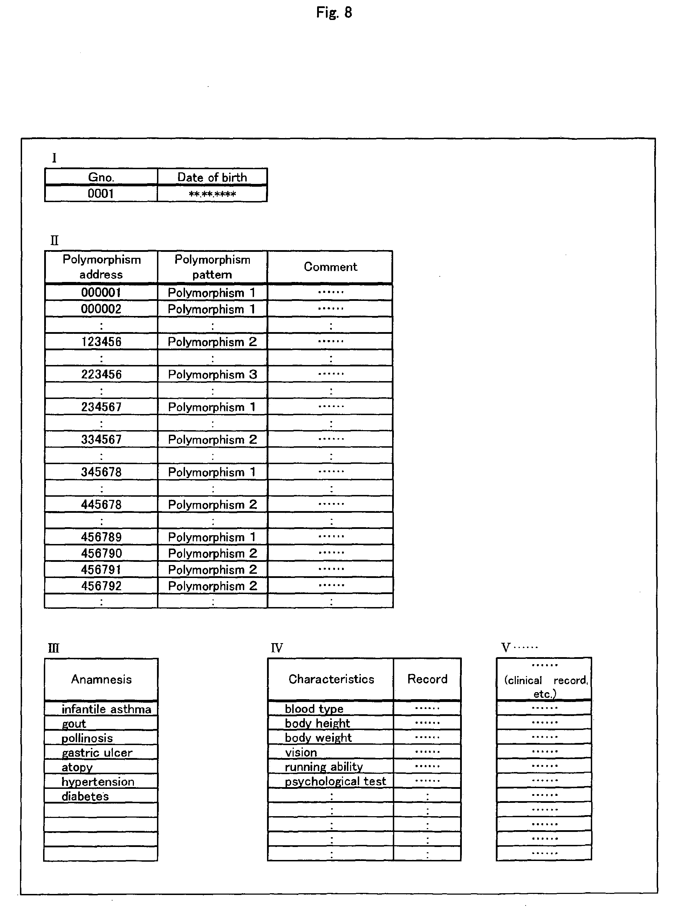

On genome-related information recording medium 24, recorded as genome-related information 28 is, for example, as shown in Fig. 8, the individual's number "Gno." (G number) peculiar to genome-related information 28 as well as the individual's information, such as date of birth, as data I; polymorphism addresses and polymorphism patterns as data II; anamnesis information as data III; characteristics as data IV; and information concerning the individual's clinical record and the like as data V. In other words, genome-related information 28 is constituted by data I, data II, data III, data IV, and data V. Data I and data II contain essential information and data III, data IV, and data V are respectively constituted by additional information.

Genome-related information 28 records a "polymorphism address" corresponding to a position in a nucleotide sequence in association with a "polymorphism pattern" in the polymorphism address. Additional information concerning a predetermined polymorphism address may be recorded as data II in association with the "polymorphism address" as a "comment." Data II may comprise all the nucleotide sequences concerning a predetermined individual organism. When all the nucleotide sequences are recorded as data II, data II comprises "polymorphism addresses" and "polymorphism patterns."

According to the present invention, personal computer 3 and genome-related information recording medium 24 are not limited to the construction as shown in Figs. 7 and 8 respectively. For example, a genome-related information recording medium may be equipped with a memory section having a processing program and a personal computer may have the genome-related information recording medium mounted thereon to operate the processing program. In this case, a personal computer can be operated in accordance with a processing program recorded in a memory section on a genome-related information recording medium.

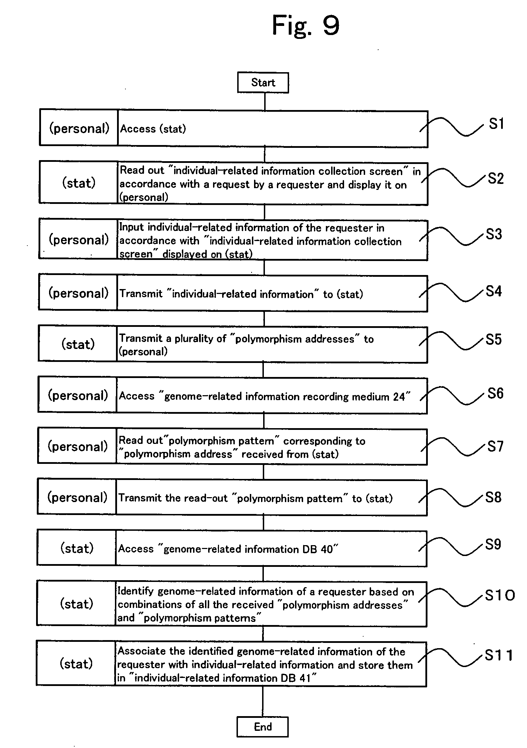

In a system for processing information having the above construction, processing program 39 of storage 33 in a computer ST for statistical analysis and processing program 27 recorded in storage 23 in personal computer 3 process information in accordance with, for example, flow chart as shown in Fig. 9. In the flow chart shown in Fig. 9, a step described as "stat" refer to process in a computer ST for statistical analysis and a step described as "individual" refer to process in personal computer 3.

According to the present system for processing information, an individual possessing genome-related information recording medium 24 uses personal computer 3 to access a computer ST for statistical analysis through communication network 1 and registers "individual-related information" concerning an individual (individual organism) in "individual-related information DB 41" of a computer ST for statistical analysis in association with genome-related information in the "genome-related information DB 40." With the present system for processing information, genome-related information recording media 24 independently comprising a plurality of genome-related information 28 of a plurality of individuals may be used, and an individual may access genome-related information recording medium 24.

In such a case, in step 1 (S1), a requester first accesses a computer ST for statistical analysis through communication network 1 and indicates the intention to register individual-related information with a computer ST for statistical analysis. In step 1, a requester may indicate one's intention by accessing a web site provided by the computer ST for statistical analysis. Alternatively, a requester may indicate one's intention by, for example, e-mailing to a computer ST for statistical analysis. In this embodiment, a case where a requester registers ones own individual-related information is described; however, it should be noted that the present invention is not limited thereto. A requester can register individual-related information concerning an individual (individual organism) other than the requester oneself.

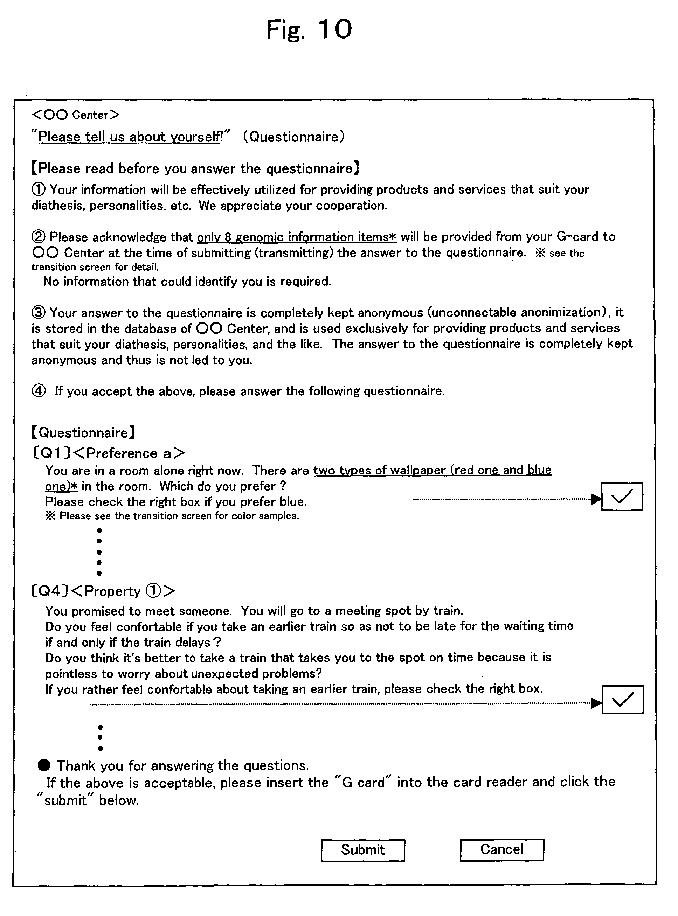

The computer ST for statistical analysis receives the indication of intention from personal computer 3, and then, in step 2 (S2), reads out an "individual-related information collection screen" as shown in Fig. 10 from screen memory 38 and displays it on display apparatus 22 of personal computer 3. In step 2, the individual-related information collection screen may be displayed in a manner such that personal computer 3 accesses the web site provided by the computer ST for statistical analysis. Alternatively, the screen may be displayed on display apparatus 22 of personal computer 3 based on the data of the individual-related information collection screen transmitted to personal computer 3.

In step 3 (S3), the requester inputs his/her own individual-related information in personal computer 3 in accordance with the individual-related information collection screen. Specifically, the requester answers a question displayed on an individual-related information collection screen and inputs the answer thereto. Alternatively, the requester may input the answer to the question displayed on the individual-related information collection screen to an answer screen, which differs from the individual-related information collection screen. In step 3, information obtained when a user (requester) or the like receives checkups at a medical, testing, or other institution may be inputted as individual-related information.

In step 4 (S4), personal computer 3 then transmits the answer (individual-related information) to the question displayed on an individual-related information collection screen to the computer ST for statistical analysis. In step 4, data displayed on the individual-related information collection screen with the answer being inputted or data displayed on the answer screen with the answer being inputted is transmitted through communication network 1. Thus, the answer to the question can be transmitted to the computer ST for statistical analysis. Alternatively, information obtained when the user (requester) or the like has received checkups at a medical or testing institution or the like can be transmitted to the computer ST for statistical analysis as individual-related information in step 4.

The computer ST for statistical analysis next receives individual-related information from personal computer 3 and then, in step 5 (S5), transmits a plurality of polymorphism addresses to personal computer 3. A plurality of polymorphism addresses to be transmitted in step 5 may be previously determined or randomly selected.

Personal computer 3 receives a plurality of polymorphism addresses from the computer ST for statistical analysis and then, in step 6 (S6), accesses genome-related information recording medium 24 by driving reading apparatus 25. Subsequently, personal computer 3 reads out polymorphism patterns corresponding to the plurality of polymorphism addresses received from the computer ST for statistical analysis in step 7 (S7). In step 8 (S8), personal computer 3 transmits the polymorphism patterns that had been read out in step 7 in association with the polymorphism addresses corresponding thereto to the computer ST for statistical analysis. More specifically, personal computer 3 transmits polymorphism patterns independently corresponding to the plurality of polymorphism addresses received from the computer ST for statistical analysis in association with the plurality of polymorphism addresses in step 8. When data is transmitted from personal computer 3 to a computer ST for statistical analysis in step 8, it is preferable to refrain from transmitting information that could specify an individual (an individual organism), such as "Gno."

In the present embodiment, personal computer 3 receives the polymorphism address transmitted from a computer ST for statistical analysis in step 5 and transmits the polymorphism pattern corresponding to the received polymorphism address to a computer ST for statistical analysis in step 8. It should be noted that the present embodiment is not limited thereto. For example, in step 4, personal computer 3 may transmit "individual-related information" to a computer ST for statistical analysis and may spontaneously transmit the predetermined polymorphism address and a polymorphism pattern corresponding thereto to a computer ST for statistical analysis. In such a case, the aforementioned steps 5 to 8 are not carried out, but step 9 and subsequent steps described below are carried out after step 4 in a similar manner.

Subsequently, a computer ST for statistical analysis receives the polymorphism address and the polymorphism pattern from personal computer 3 and then accesses genome-related information DB 40 in step 9 (S9). In the case that genome-related information DB 40 is possessed by an external organization instead of the computer ST for statistical analysis, personal computer 3 accesses genome-related information DB 40 of the external organization through communication network 1 in step 9.

In step 10 (S10), the computer ST for statistical analysis searches genome-related information DB 40 based on a plurality of combinations of polymorphism addresses and polymorphism patterns received from personal computer 3 and identifies genome-related information concerning the individual (an individual organism) having the received plurality of combinations of polymorphism addresses and polymorphism patterns from among genome-related information stored in the genome-related information DB 40. In other words, in step 10, genome-related information concerning the requester (the individual (individual organism) related to the "individual-related information" transmitted by the requester) is identified from among a plurality of genome-related information concerning a plurality of individuals (individual organisms) registered in genome-related information DB 40. In step 10, for example, a "reference No." may be applied to genome-related information concerning an identified requester, i.e., an individual (individual organism). Alternatively, in step 10, a "reference No." that has been previously applied to the genome-related information concerning the individual (individual organism) who has been registered in genome-related information DB 40 may be extracted.

In step 11 (S11), the computer ST for statistical analysis allows the genome-related information concerning the requester identified in step 10 (the individual (individual organism) related to the "individual-related information" transmitted by the requester) to associate with the individual-related information received from personal computer 3. Specifically, the "individual-related information DB 41" as shown in Fig. 6 in which the "reference No." applied to the genome-related information concerning the requester (the individual (individual organism) related to the "individual-related information" transmitted by the requester) identified in step 10 or the "reference No." extracted concerning the genome-related information concerning the requester (the individual (individual organism) related to the "individual-related information" transmitted by the requester) identified in step 10 is stored in association with individual-related information is constructed.

Alternatively, in step 10, in the case that the computer ST for statistical analysis possesses genome-related information DB 40, individual-related information received from personal computer 3 may be stored in association with the genome-related information concerning the requester (the individual (individual organism) related to the "individual-related information" transmitted by the requester) stored in genome-related information DB 40 directly.

According to the process of the flow chart shown in Fig. 9, for example, individual-related information concerning a predetermined requester can be registered in the computer ST for statistical analysis in association with the genome-related information of the requester. If more than one requester independently register their individual-related information in accordance with the process of the flow chart shown in Fig. 9, the computer ST for statistical analysis can possess a plurality of individual-related information concerning the plurality of individuals (individual organisms). More specifically, the computer ST for statistical analysis can construct a database storing genome-related information concerning a plurality of individuals in association with individual-related information.

Accordingly, even when individual-related information that is anonymized later is transmitted to the computer ST for statistical analysis corresponding to the genome-related information that has been previously anonymously recorded in "genome-related information DB 40" of the computer ST for statistical analysis, the genome-related information can be linked to the individual-related information with the utilization of the present system.

According to the process of the flow chart shown in Fig. 9, a computer ST for statistical analysis receives polymorphism patterns concerning a plurality of polymorphism addresses transmitted in step 5 from personal computer 3 and searches the genome-related information DB 40 in step 10 to identify the genome-related information concerning the requester (the individual (individual organism) related to the "individual-related information" transmitted by the requester). In the present system for processing information, however, a method for identifying the genome-related information concerning the requester (the individual (individual organism) related to the "individual-related information" transmitted by the requester) is not limited thereto. For example, a combination of a predetermined "polymorphism address" and a "polymorphism pattern" may be successively transmitted from personal computer 3 to a computer ST for statistical analysis to allow the computer ST for statistical analysis to identify the requester. In this case, a combination of a predetermined "polymorphism address" and a "polymorphism pattern" may be successively and spontaneously transmitted from personal computer 3 to a computer ST for statistical analysis. Alternatively, a computer ST for statistical analysis may successively request personal computer 3 to submit the "polymorphism pattern" corresponding to a predetermined "polymorphism address," and personal computer 3 may successively transmit the "polymorphism pattern" corresponding to the request in association with the "polymorphism address."

More specifically, a computer ST for statistical analysis repeats a step of receiving one or more combinations of "polymorphism address" and "polymorphism pattern" concerning the requester and a step of searching for the genome-related information concerning an individual having a combination of a "polymorphism address" and a "polymorphism pattern" that matches the received combination of "polymorphism address" and "polymorphism pattern" from genome-related information DB 40 until the genome-related information concerning one individual (individual organism) is consequently identified as the genome-related information concerning the requester (the individual (individual organism) related to the "individual-related information" transmitted by the requester). Thus, the genome-related information concerning a predetermined individual (individual organism) contained in genome-related information DB 40 can be identified as genome-related information concerning the requester (the individual (individual organism) related to the "individual-related information" transmitted by the requester).

The computer ST for statistical analysis uses individual-related information DB 41 storing a plurality of individual-related information concerning a plurality of individuals in association with the genome-related information contained in genome-related information DB 40 to statistically process such information. Thus, semantic information implied by the nucleotide sequence-related information and/or information associated with the semantic information can be created.

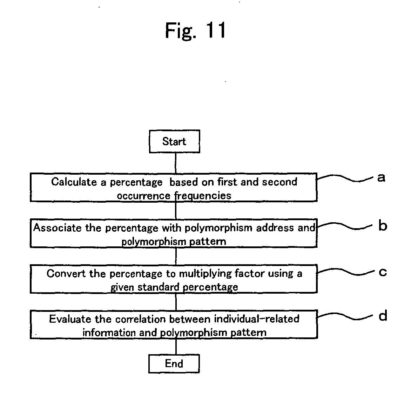

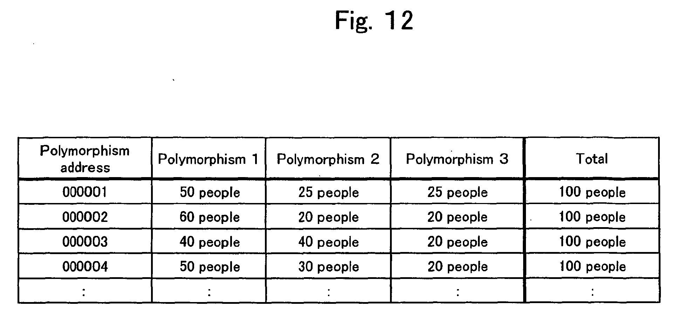

For example, a statistical process can be carried out in accordance with the flow chart shown in Fig. 11. At the outset, an occurrence frequency of a polymorphism pattern (the first occurrence frequency) that can appear in each polymorphism address is calculated based on all the data contained in genome-related information DB 40 (all the data associated with all "reference Nos.") at that time. As shown in Fig. 12, for example, the result of calculation is represented by a matrix comprising the polymorphism addresses arranged in rows and the occurrence frequencies of polymorphism patterns arranged in columns. According to Fig. 12, "polymorphism 1," i.e., the polymorphism pattern in the polymorphism address "000001," is expressed in 50 out of 100 individuals.

Genome-related information DB 40 and individual-related information DB 41 are then used to calculate the occurrence frequency of a polymorphism pattern (the second occurrence frequency) that can appear in each polymorphism address concerning predetermined individual-related information.

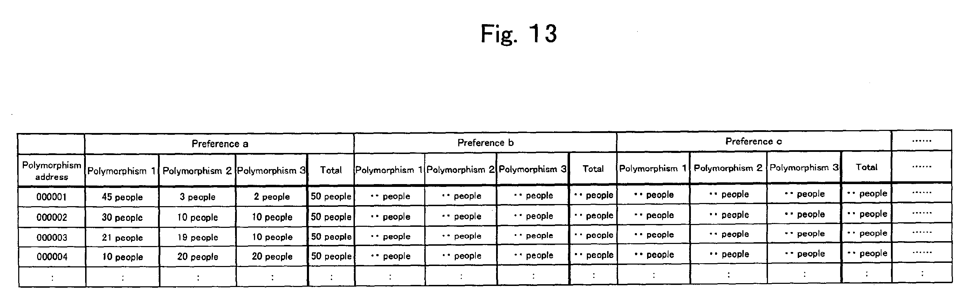

Specifically, a "reference No." with a property relevant to, for example, "preference a (e.g., a preference for blue over red)," is selectively extracted (having "○" in the result column) from individual-related information DB 41, the extracted "reference No." is searched for from genome-related information DB 40, and the occurrence frequency of each polymorphism pattern that can appear in each polymorphism address is then calculated based on the searched data. The results of calculation are represented by a matrix comprising polymorphism addresses arranged in rows and occurrence frequency of polymorphism patterns separately arranged in columns for each individual-related information (for each "preference"), for example, as shown in Fig. 13. According to Fig. 13, when the "reference No." has a property relevant to "preference a," "polymorphism 1," which is the polymorphism pattern in the polymorphism address "000001," is expressed in 45 out of 50 individuals.

Using the results of the occurrence frequencies shown in Fig. 12 (the first occurrence frequency) and the occurrence frequencies shown in Fig. 13 (the second occurrence frequency), the occurrence frequency (Fig. 12) of a polymorphism pattern that can appear in each polymorphism address that is calculated regardless of relevance to a given piece of individual-related information, and the occurrence frequency (Fig. 13) of a polymorphism pattern that can appear in each polymorphism address that is calculated only when the "reference No." has a property relevant to the given piece of individual-related information are compared to determine relative values in step (a).

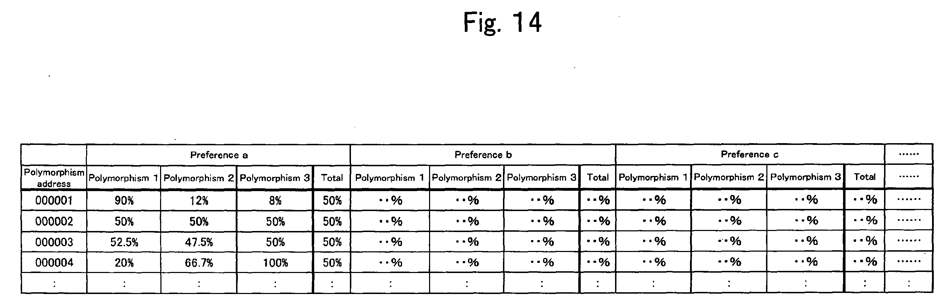

A specific example thereof is as follows. The polymorphism pattern "polymorphism 1" in the polymorphism address "000001" is occurred in 45 out of 50 individuals when the "reference No." has a relevant property concerning "preference a" (which is shown in Fig. 13). This value 45 is divided by 50, which is the number out of 100 individuals in which the frequency of polymorphism pattern "polymorphism 1" is occurred in the polymorphism address "000001" among all the data contained in genome-related information DB 40 at that time (all the data associated with all the "reference Nos.," which is shown in Fig. 12). The thus obtained percentage (90%) is determined as the relative value. Similarly, the percentage (12%) in the case of the polymorphism pattern "polymorphism 2" in the polymorphism address "000001" and the percentage (8%) in the case of the polymorphism pattern "polymorphism 3" in the polymorphism address "000001" are determined. The results are, for example, represented by a matrix comprising polymorphism addresses of individual-related information separately arranged in rows and percentages of each polymorphism pattern separately arranged in columns as shown in Fig. 14 (step (b)).

If "preference a" is not affected at all by differences in polymorphism patterns in the polymorphism address "000001," the percentages (relative values) calculated above should be comparable with each other among "polymorphism 1," "polymorphism 2," and "polymorphism 3." Statistically, this predisposition is known to more significantly appear when the parameter for calculating the occurrence frequency as shown in Figs. 12 and 13 is larger.

When determining the results shown in Fig. 14, for example, data indicating the occurrence frequency of each polymorphism pattern that can appear in the polymorphism address calculated (statistically processed) by an external organization other than the computer ST for statistical analysis or the original data for calculating the occurrence frequency may be used instead of the occurrence frequency shown in Fig. 12. In such a case, individuals associated with the individual-related information contained in individual-related information DB 41 may or may not be included in individuals associated with the genome-related information, which has been used by the external organization for determining the occurrence frequency.

A method for determining the percentages is not limited to the aforementioned method, and other methods of calculation may be employed.

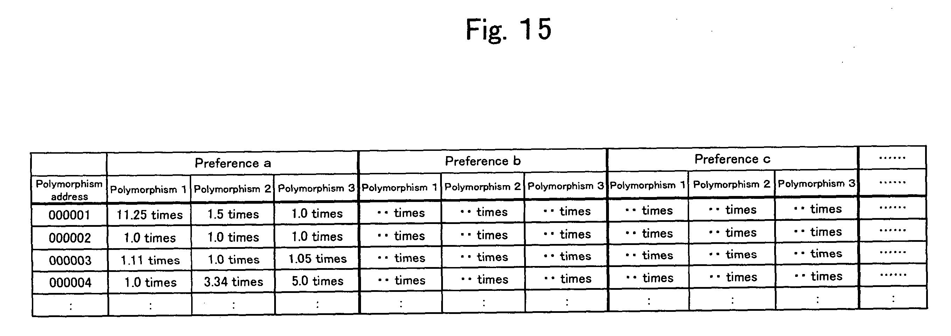

Subsequently, the percentages shown in Fig. 14 are used in step (c) to calculate relative values for each polymorphism pattern that can appear in each polymorphism address. In the case of the results shown in Fig. 14, for example, the percentage of the polymorphism pattern "polymorphism 1" in the polymorphism address "000001" is denoted as 90%, the percentage of the polymorphism pattern "polymorphism 2" is denoted as 12%, and the percentage of the polymorphism pattern "polymorphism 3" is denoted as 8%, concerning "preference a." The percentage (8%) of the polymorphism pattern "polymorphism 3" that represents the minimal percentage is employed as the standard, and the percentage of each polymorphism pattern is divided by the percentage (8%) of "polymorphism 3." More specifically, the relative value of the polymorphism pattern "polymorphism 1" is 11.25 (=90/8), the relative value of the "polymorphism 2" is 1.5 (=12/8), and the relative value of the "polymorphism 3" is 1.0 (=8/8). The percentage of the "polymorphism 3" representing the minimal percentage is employed as the standard to determine the relative value for each polymorphism pattern. The results thereof are represented by a matrix comprising polymorphism addresses of individual-related information separately arranged in rows and relative values for each polymorphism pattern arranged in columns as shown in, for example, Fig. 15.

If "preference a" is not affected at all by differences in polymorphism patterns in the polymorphism address "000001," the multiplying factor (relative value) calculated above of "polymorphism 1," that of "polymorphism 2," and that of "polymorphism 3" should be close to 1.0. Statistically, this predisposition is known to more significantly appear when the parameter for calculating the occurrence frequency as shown in Figs. 12 and 13 is larger.

When the relative value exceeds a predetermined level, differences in polymorphism patterns in the polymorphism address representing the relative value are evaluated to be involved with (whether or not such differences are related to a property relevant to) predetermined individual-related information based on the results shown in Fig. 15 in step (d). A specific example thereof is as follows. When the multiplying factor (relative value) of the result shown in Fig. 15 exceeds the threshold value, the polymorphism address indicating the multiplying factor and the multiplying factor thereof are extracted and outputted. The threshold value can be statistically calculated by surveying all the relative values if applicable to, for example, predetermined individual-related information as shown in Fig. 15.

Depending on the type of a method for calculating the relative values as shown in Fig. 15, when the relative value is below a predetermined level, differences in polymorphism patterns in the polymorphism address indicating the relative value may be evaluated to be involved with (whether or not such differences are related to a property relevant to) predetermined individual-related information.

As the extracted multiplying factor (relative value) becomes relatively larger (i.e., as the absolute value of a difference between the multiplying factor (relative value) and the threshold (predetermined standard value) becomes larger), differences in polymorphism patterns in the polymorphism address indicating the extracted multiplying factor can be deduced to be deeply involved with (whether or not such differences are related to a property relevant to) predetermined individual-related information. Specifically, an inherent (genetic) factor is deduced to be deeply involved with (whether or not such differences are related to a property relevant to) predetermined individual-related information.

When simultaneous parallel processes of attaining the results shown in Fig. 15 from the occurrence frequencies shown in Figs. 12 and 13 are carried out concerning a plurality of individual-related information, which individual-related information have relevance to each other can be deduced based on a tendency of the each polymorphism addresses and the multiplying factors thereof (relative values) extracted concerning a plurality of individual-related information.

If the results shown in Fig. 15 are attained from the occurrence frequencies shown in Figs. 12 and 13 and evaluation is made based thereon, whether or not the individual-related information, on which inherent (genetic) influence has been considered to be slight, is genetically influenced can be inspected in accordance with the process shown in the flow chart shown in Fig. 11. Specifically, use of various types of individual-related information obtained from each individual (individual organism) enables estimation of the correlation between heredity and personal properties or the like, where the degree of inherent (genetic) influence on such properties has been considered to be slight in the past.

In the process of attaining the results shown in Fig. 15 from the occurrence frequencies shown in Figs. 12 and 13 and conducting evaluation based thereon, the occurrence frequency of each polymorphism pattern that is calculated regardless of relevance to individual-related information (attribution) is employed as a parameter (Fig. 12). Thus, this process is suitable for quantitatively representing the degree of genetic influence on individual-related information (attribution). In contrast, a group of individuals having properties relevant to individual-related information (attribution) is compared with a group thereof irrelevant thereto in common techniques of analyzing correlation. Thus, it is difficult to indicate the degree of genetic influence when the individual-related information (attribution) is relatively insusceptible to genetic influence, such as that regarding personalities. Specifically, in the case of the individual-related information (attribution) such as personalities, relevance to the individual-related information (attribution) is difficult to evaluate due to the desires of each individual, for example, the type of person a person wants to be. Thus, accurate comparison is difficult because of the possibility that the parameters contain errors.

Concerning the results shown in Fig. 15, when the multiplying factor (relative value) exceeds the threshold value, and the polymorphism address indicating the multiplying factor and the multiplying factor are extracted, the occurrence frequencies and the percentages shown in Figs. 12, 13, and 14 and the original numerical values used for calculating such values are preferably extracted and outputted together. In such a case, the occurrence frequencies and the percentages shown in Figs. 12, 13, and 14 and the original numerical values used for calculating such values can be used by the user of the results shown in Fig. 15 as material for evaluating the credibility of data.

Thereafter, a computer ST for statistical analysis uses the polymorphism address extracted based on the results shown in Fig. 15 and the multiplying factor thereof, and/or the original numerical values used for calculating such values that have been extracted together. Thus, knowledge, such as the correlation between predetermined individual-related information and predetermined polymorphism address and differences in the influence on predetermined individual-related information caused by the types of polymorphism patterns, can be found. The computer ST for statistical analysis can create semantic information and/or information associated with the semantic information based on the knowledge. For example, the "multiplying factor" extracted from the results shown in Fig. 15 can be used as semantic information in that state.

The computer ST for statistical analysis can construct a database (hereafter referred to as "reference DB") that sequentially records therein semantic information created based on the result of the calculated multiplying factor (relative value) as shown in Fig. 15 and/or the knowledge derived from the results of calculation as shown in Fig. 15 and/or information associated with the semantic information.

Even when the correlation between predetermined individual-related information (properties) and predetermined polymorphism address cannot be found, the correlation between a combination of a piece of or a plurality of other individual-related information (properties) and predetermined polymorphism address can be found in the following manner.

For example, it is assumed that no polymorphism address that is correlated with a piece of individual-related information (properties), i.e., "hyperhidrosis," could be found. Properties related to the property "hyperhidrosis," such as "frequent fluid intake" and "preference for salty food," are assumed to be correlated with "polymorphism address 000001" and "polymorphism address 000101," respectively. The correlation between a predetermined property and other properties refers to the fact that a probability of an individual with the predetermined property exhibiting the other properties is at a predetermined level or higher, such as 80% or higher. The correlation can be derived from, for example, the results of questionnaires concerning the predetermined property and the other properties. When 80% or more respondents to the questionnaires who answered that they had the predetermined property also had the other properties, for example, existence of a correlation between the given property and other properties can be presumed.

It is further assumed that 80% of the individuals having the property "frequent fluid intake" have "polymorphism 1" in the "polymorphism address 000001" and 70% of individuals having the property "preference for salty foods" have "polymorphism 2" in the "polymorphism address 000101."

Given the circumstances, the property "hyperhidrosis" can be found to be indirectly correlated with the "polymorphism address 000001" and the "polymorphism address 000101." Specifically, it can be indirectly found that 56% (0.8 x 0.7 = 0.56) of the individuals having "polymorphism 1" in the "polymorphism address 000001" and "polymorphism 2" in "polymorphism address 000101" exhibit the property "hyperhidrosis."

When an individual has "polymorphism 1" in the "polymorphism address 000001" and "polymorphism 2" in "polymorphism address 000101," accordingly, semantic information such as "predisposition to hyperhidrosis (e.g., "index 56")" can be created. Further, information associated with the semantic information, such as "points to be concerned about regarding lifestyle," can be derived from the created semantic information.

Thus, the computer ST for statistical analysis can provide the created semantic information and/or information associated with the semantic information to shared computer 2. Shared computer 2 can construct main DB 14 based on the semantic information provided by the computer ST for statistical analysis or a database for providing information based on the information associated with the semantic information provided by the computer ST for statistical analysis.

The computer ST for statistical analysis constructs "reference DB" by sequentially recording the created semantic information and/or information associated with the semantic information as mentioned above therein to use this "reference DB" as main DB 14. Thus, it can function as shared computer 2.

Shared computer 2 can use the constructed main DB 14 to provide semantic information, such as the morbidity rate of a given disease, to users in the following manner. Specifically, shared computer 2 can provide semantic information, such as the morbidity rate of a given disease, when, for example, the user intends to learn of his/her own morbidity rate of the given disease as "a request for an object and/or service."

In the present system for processing information, "an object and/or service" is not limited to the morbidity rate of a given disease. Examples thereof include objects such as pharmaceutical products, foods, and nonessential grocery items that suit individuals' (individual organisms') diatheses and services such as information that suits individuals' (individual organisms') diatheses and properties.

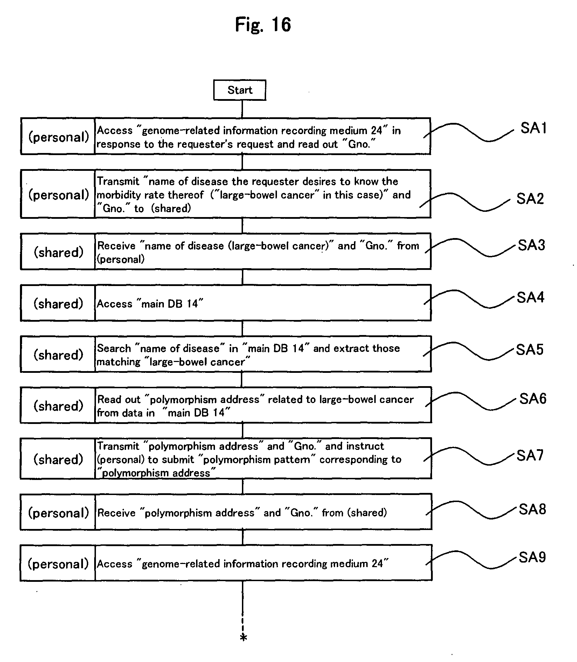

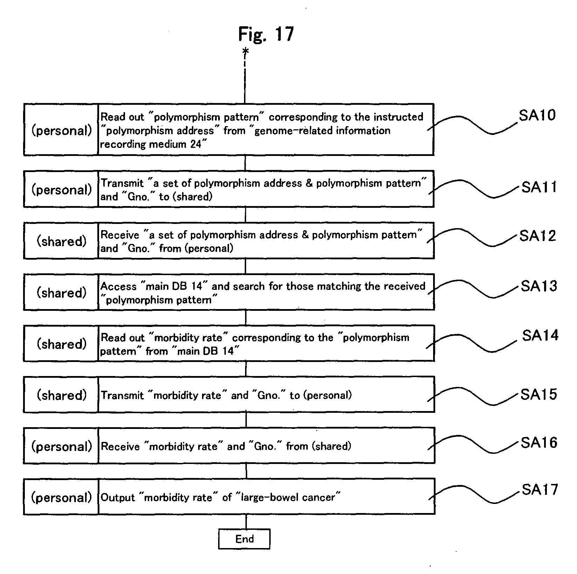

In a system for processing information wherein shared computer 2 provides a morbidity rate of a given disease to the user, processing program 13 recorded in storage 7 in shared computer 2 and processing program 27 recorded in storage 23 in personal computer 3 process information in accordance with, for example, flow charts as shown in Figs. 16 and 17. In the flow charts as shown in Figs. 16 and 17, a step described as "(shared)" refers to processing in shared computer 2 and a step described as "(personal)" refers to processing in personal computer 3.

The system for processing information is a system in which an individual possessing genome-related information recording medium 24 accesses shared computer 2 using personal computer 3 through communication network 1 and utilizes semantic information recorded in main DB 14 in shared computer 2. The system for processing information may be a system comprising the genome-related information recording medium 24, having genome-related information 28 on a plurality of individuals recorded thereon, to which individuals respectively access.

In this case, when utilizing the system, the requester first starts processing program 27, which is recorded in storage 23, in step A1 (SA1). Processing program 27 drives reading apparatus 25 in personal computer 3 to access genome-related information recording medium 24. Thus, "Gno." recorded as data I on genome-related information recording medium 24 is read out and the read-out "Gno." is stored in memory section 26.

In step A2 (SA2), based on a screen image displayed by processing program 27 on display device 22, information, the provision of which is desired by the requester wishes to receive, for example, the "morbidity rate of large-bowel cancer" (requested information), is input to personal computer 3. At the same time, the "morbidity rate of large-bowel cancer" and "Gno." are transmitted to shared computer 2 from personal computer 3 through communication network 1. Alternatively, the requester writes the "morbidity rate of large-bowel cancer" and "Gno." in shared computer 2 from personal computer 3 through communication network 1.

In step A3 (SA3), shared computer 2 receives the "morbidity rate of large-bowel cancer" and "Gno." The received "morbidity rate of large-bowel cancer" and "Gno." are stored in memory section A10 as request information.

In step A4 (SA4), upon the reception of request information, processing program 13 recorded in storage 7 is started to access main DB 14. This processing program 13 performs processing in shared computer 2.

In step A5 (SA5), in accordance with processing program 13, "classification (name of disease)" recorded in main DB 14 is searched and information matching with the requested "morbidity rate of large-bowel cancer" (large-bowel cancer) is extracted.

In step A6 (SA6), from among data recorded in main DB 14, a "polymorphism address" associated with "classification (name of disease)" (large-bowel cancer) that matches with the "morbidity rate of large-bowel cancer" is read out. The read-out "polymorphism address" is stored as positional information associated with request information in memory section A10. Specifically, the "morbidity rate of large-bowel cancer" and "polymorphism address" are recorded in memory section A10 in association with a predetermined "Gno."

In step A7 (SA7), "Gno." and "polymorphism address" recorded in memory section A10 are transmitted to personal computer 3 and instruction information instructing submission of a "polymorphism pattern" corresponding to the transmitted "polymorphism address" is transmitted to personal computer 3. At this time, the submission of additional information such as that concerning anamnesis and characteristics may be optionally instructed depending on the types of request information.

In step A8 (SA8), "Gno.," "polymorphism address," and instruction information transmitted from shared computer 2, are received. The received "Gno." and "polymorphism address" are recorded in memory section 26.

In step A9 (SA9), data II recorded on genome-related information recording medium 24 is accessed in accordance with the received instruction information. In step A10 (SA10), in accordance with processing program 27, data II recorded on genome-related information recording medium 24 is searched, a polymorphism pattern in the instructed polymorphism address is read out; and the polymorphism pattern is then recorded in memory section 26 in association with the polymorphism address. In this case, whether the "Gno." received in step A8 is correct or not is preferably confirmed by accessing data 1. In step A10, additional information recorded in data III, data IV, and data V is read out simultaneously with the polymorphism pattern and may be optionally recorded in memory section 26.

In step A11 (SA11), the temporarily-recorded polymorphism pattern associated with the polymorphism address and the optionally-recorded additional information in memory section 26 are output to shared computer 2 together with "Gno." through communication network 1. In step A12 (SA12), shared computer 2 receives the polymorphism pattern associated with the polymorphism address and the optionally-recorded additional information, and the received polymorphism pattern is recorded in memory section A10 in association with the polymorphism address.

In this embodiment, in step A7 shared computer 2 transmits instruction information instructing submission of the "polymorphism pattern", and in step A10 personal computer 3 reads out the polymorphism patterns from genome-related information recording medium 24 in accordance with instruction information. The system, however, may not transmit the instruction information in step A7. In this case, personal computer 3 searches data II in step A10 based on the polymorphism address received in step A8 and reads out polymorphism patterns of the received polymorphism address in accordance with processing program 27. Then, in step A11, personal computer 3 outputs polymorphism patterns and the like to shared computer 2. Also in this case, in step A12 shared computer 2 can obtain the polymorphism pattern of the "polymorphism address" associated with "classification (name of disease)" that matches the "morbidity rate of large-bowel cancer".

In step A13 (SA13), main DB 14 is accessed to search information matching with the received polymorphism address and polymorphism patterns. More specifically, a plurality of polymorphism patterns are recorded in main DB 14 for one polymorphism address. Thus, which polymorphism pattern in main DB 14 matches with the received polymorphism address and the polymorphism pattern thereof is searched.

In step A14 (SA14), the morbidity rate of large-bowel cancer which is associated with the polymorphism pattern matching the received polymorphism pattern is read out in accordance with processing program 13. Specifically, in step A14, the morbidity rate of large-bowel cancer of a requester can be read out in accordance with the polymorphism address and polymorphism pattern submitted by the requester. The read-out morbidity rate is stored in memory section A10 in association with the requester's "Gno." At this time, the morbidity rate of large-bowel cancer may be corrected in accordance with additional information and then stored. Alternatively, other information obtained from additional information may be stored in association with the requester's "Gno."

Subsequently, in step A15 (SA15), the requester's "Gno." and morbidity rate, which are stored in memory section A10, are transmitted as semantic information to personal computer 3 through communication network 1. Personal computer 3 receives the requester's "Gno." and morbidity rate (semantic information) in step A16 (SA16). The received semantic information is recorded in memory section 26.

In step A17 (SA17), the morbidity rate of large-bowel cancer is displayed on display device 22 based on semantic information recorded in memory section 26 in accordance with processing program 27. Instead of steps A15 to A17, shared computer 2 can read out (prepare) a screen that displays semantic information in accordance with processing program 13, and display it on display device 22 of personal computer 3 through communication network 1. Also in this case, semantic information is considered to be transmitted from shared computer 2 to personal computer 3. As a result, the requester can obtain the morbidity rate of large-bowel cancer using genome-related information 28 recorded on genome-related information recording medium 24.

As described above, in this system, utilization of genome-related information recording medium 24, which has individuals' polymorphism patterns in association with polymorphism addresses recorded thereon, enables individuals to use semantic information recorded in main DB 14 through the polymorphism addresses. In other words, an individual utilizing this system does not have to record semantic information on a genome-related information recording medium. Instead, the individual can obtain various semantic information simply by possessing genome-related information 28 having the polymorphism pattern associated with the polymorphism address.

More particularly, as described above, semantic information is corrected and increases in the number of types. Thus, by updating main DB 14 the semantic information becomes more accurate and includes a wider range of information. According to this system, individuals can utilize the newest semantic information by updating main DB 14 in line with such increase, correction or the like of semantic information.

Further, utilization of genome-related information recording medium 24 having genome-related information 28 recorded thereon eliminates the need for the user to undergo an examination to obtain genome-related information every time he/she utilizes this system. More specifically, once genome-related information recording medium 24 is produced, thereafter the user can obtain the newest semantic information utilizing this system.

Retention by the user of genome-related information recording medium 24 on which the user's genome-related information 28 has been recorded can prevent apprehension in consigning an external organization to store the user's genome-related information 28 and the risk of leakage of genome-related information 28 through unauthorized access to the organization. On the other hand, when storage of genome-related information recording medium 24 having a plurality of pieces of genome-related information 28 of a plurality of individuals recorded thereon is consigned to an external organization, inefficient handling of genome-related information recording medium 24 or loss of genome-related information recording medium 24 can be prevented, unlike the case where each individual retains their own genome-related information recoding medium 24.

In particular, in accordance with the flow charts shown in Figs. 16 and 17, all the genome-related information 28 recorded on genome-related information recording medium 24 is not necessarily output through communication network 1, and only the part of genome-related information 28 for which an instruction for the submission was received may be output. According to this system, leakage of highly confidential polymorphism addresses and polymorphism patterns peculiar to individuals can be prevented.

Also, in accordance with the flow charts shown in Figs. 16 and 17, personal computer 3 does not need to handle information recorded in main DB 14 since shared computer 2 obtains semantic information to provide to the requester. Thus, in accordance with the flow charts shown in Figs. 16 and 17, desired semantic information can be adequately obtained even if the capacity of personal computer 3 to process information is relatively low. Further, since personal computer 3 does not need to handle information recorded in main DB 14, standardization of processing program 27 in personal computer 3 in compliance with a card drive and the like having genome-related information recording medium 24 mounted thereon is facilitated.

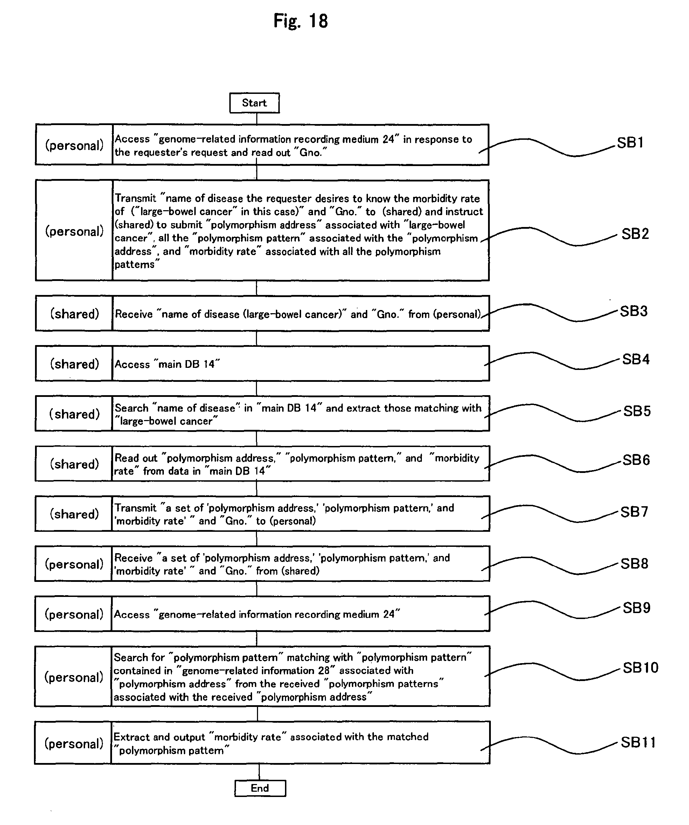

In the system for processing information, processing program 13 recorded in storage 7 of shared computer 2 and processing program 27 recorded in storage 23 of personal computer 3 may process information in accordance with, for example, the flow chart shown in Fig. 18. Also in the flow chart shown in Fig. 18, a step described as "(shared)" refers to processing in shared computer 2 and a step described as "(personal)" refers to processing in personal computer 3.

When utilizing the system, the requester first starts processing program 27 recorded in storage 23 in step B1 (SB1). Processing program 27 drives reading apparatus 25 in personal computer 3 and accesses genome-related information recording medium 24, thereby reading out a "Gno." recorded in genome-related information recording medium 24 as data I. The read-out "Gno." is stored in memory section 26.

In step B2 (SB2), based on a screen image displayed by processing program 27 on display device 22, information which the requester wishes to receive, for example, the "morbidity rate of large-bowel cancer" (request information), is input to personal computer 3. At the same time, the "morbidity rate of large-bowel cancer" and "Gno." are transmitted to shared computer 2 from personal computer 3 through communication network 1. Concurrently requested is the submission of "polymorphism address" in which "classification (name of disease)" in main DB 14 is large-bowel cancer, all the "polymorphism patterns" associated with the "polymorphism address," and the "morbidity rate" implied by all the "polymorphism patterns" respectively. More specifically, in step B2, the requester requests information comprising the "polymorphism address" in which "classification (name of disease)" in main DB 14 is large-bowel cancer, all the "polymorphism patterns" associated with the "polymorphism address," and the "morbidity rate" implied by all the "polymorphism patterns" respectively.

In step B3 (SB3), shared computer 2 receives the request information. Shared computer 2 starts processing program 13 upon reception of the request information and, in step B4 (SB4), accesses main DB 14 in accordance with processing program 13.

In step B5 (SB5), in accordance with processing program 13, "classification (name of disease)" recorded in main DB 14 is searched and information matching with the requested "morbidity rate of large-bowel cancer" (large-bowel cancer) is extracted. In step B6 (SB6), main DB 14 is accessed according to processing program 13 to read out the "polymorphism address" associated with "classification (name of disease)" (large-bowel cancer) that matches the "morbidity rate of large-bowel cancer," all the "polymorphism patterns" associated with the polymorphism address, and the "morbidity rate" in all the polymorphism patterns. The read-out "polymorphism address," "polymorphism pattern," and "morbidity rate" are stored in memory section A10 in association with the request information. Specifically, the "polymorphism address," the "polymorphism pattern," and the "morbidity rate" are recorded in memory section A10 for a predetermined "Gno."

In step B7 (SB7), the "Gno." "polymorphism address," "polymorphism pattern," and "morbidity rate" recorded in memory section A10 are transmitted to personal computer 3 through communication network 1. In step B8 (SB8), the "Gno." "polymorphism address," "polymorphism pattern," and "morbidity rate" transmitted from the shared computer 2 are received. The received "Gno." "polymorphism address," "polymorphism pattern," and "morbidity rate" are recorded in memory section 26.

In step B9 (SB9), data II, recorded on genome-related information recording medium 24, is accessed according to processing program 27. In this case, data I, recorded on genome-related information recording medium 24, is also accessed and whether the received "Gno." is correct or not is preferably confirmed.

In step B10 (SB10), the polymorphism pattern in the polymorphism address matching with the received "polymorphism address" is extracted from genome-related information 28 according to processing program 27. In step B10 (SB10), polymorphism pattern matching with the extracted polymorphism pattern is searched from all the received "polymorphism patterns" associated with the received polymorphism address.

In step B11 (SB 11), the "morbidity rate" associated with the matching polymorphism pattern among all the received "polymorphism patterns" associated with the received polymorphism address is extracted and the extracted "morbidity rate" is output. This enables the requester to obtain the morbidity rate of large-bowel cancer (semantic information). In step B11, additional information recorded in data III, data IV, and data V is simultaneously read out and the morbidity rate of large-bowel cancer may be corrected by the additional information and then output.

More specifically, in accordance with the flow chart shown in Fig. 18, genome-related information 28 recorded on genome-related information recording medium 24 is output only to personal computer 3 and other than this is not output at all to the outside. That is, genome-related information 28 is transmitted/received only between genome-related information recording medium 24 and personal computer 3. Thus, the system can more accurately prevent leakage of highly confidential genome-related information 28 peculiar to the individual.

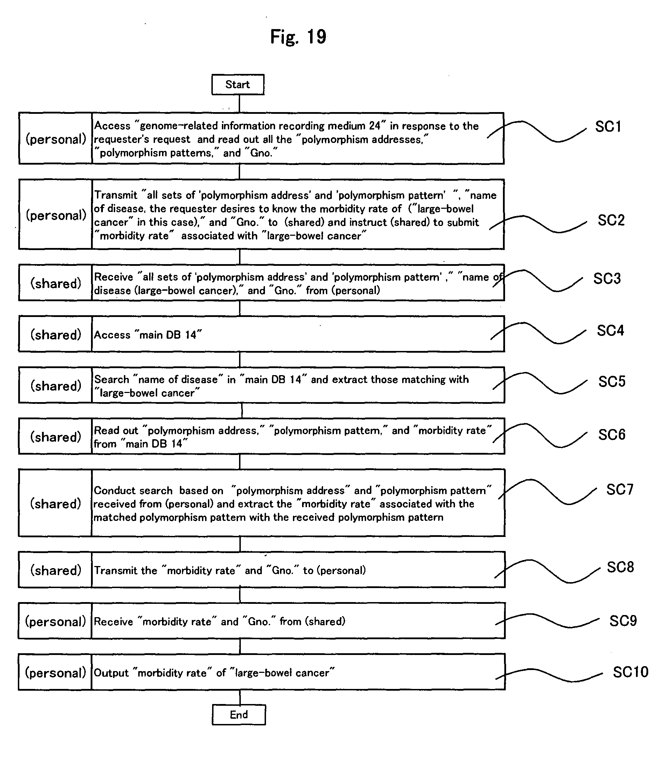

In the system for processing information, processing program 13 recorded in storage 7 of shared computer 2 and processing program 27 recorded in storage 23 of personal computer 3 may process information, for example, in accordance with the flow chart shown in Fig. 19. In the flow chart shown in Fig. 19, a step described as "(shared)" refers to processing in shared computer 2 and a step described as "(personal)" refers to processing in personal computer 3.

When utilizing the system, the requester first starts processing program 27 recorded in storage 23 in step C1 (SC1). Processing program 27 drives reading apparatus 25 in personal computer 3 and accesses genome-related information recording medium 24 to read out a "Gno." recorded therein as data I and all "polymorphism addresses" and "polymorphism patterns" recorded therein as data II. The read-out "Gno.", "polymorphism address", and "polymorphism pattern" are stored in memory section 26.

In step C2 (SC2), based on a screen image displayed by processing program 27 on display device 22, information which the requester wishes to receive, for example the "morbidity rate of large-bowel cancer" (request information), is input to personal computer 3. At the same time, the "morbidity rate of large-bowel cancer" and "Gno.", "polymorphism address," and "polymorphism pattern" recorded in memory section 26 are transmitted to shared computer 2 from personal computer 3 through communication network 1.

In step C3 (SC3), shared computer 2 receives "morbidity rate of large-bowel cancer," "Gno.", "polymorphism address," and "polymorphism pattern." The received "morbidity rate of large-bowel cancer" is recorded as request information in memory section A10 and "Gno.", "polymorphism address" and "polymorphism pattern" are also stored in memory section A10. Shared computer 2 starts processing program 13 upon reception of the request information and, in step C4 (SC4), accesses main DB 14 in accordance with processing program 13.

In step C5 (SC5), in accordance with processing program 13, "classification (name of disease)" recorded in main DB 14 is searched and classification matching with the requested "morbidity rate of large-bowel cancer" (large-bowel cancer) is extracted.

In step C6 (SC6), main DB 14 is accessed in accordance with processing program 13 to read out from main DB 14 the "polymorphism address" classified into "large-bowel cancer", all the "polymorphism patterns" associated with the polymorphism address, and the "morbidity rate" in all the polymorphism patterns. The read-out "polymorphism address," "polymorphism pattern," and "morbidity rate" are stored in memory section A10.

In step C7 (SC7), the data stored in memory section A10 in step C6 is searched based on the "polymorphism address" and the "polymorphism pattern" received in step C3, and a morbidity rate associated with polymorphism pattern matching with the received "polymorphism pattern" is extracted from memory section A10.

In step C8 (SC8), the result of step C7, that is, the morbidity rate extracted according to which polymorphism pattern in main DB 14 matches with the polymorphism pattern contained in the received information in step C3, is transmitted to personal computer 3 through communication network 1. In this case, shared computer 2 transmits the extracted morbidity rate together with the requester's "Gno."

In step C9 (SC9), the "Gno." and "morbidity rate (semantic information)" transmitted from shared computer 2 is received. The received "Gno." and "morbidity rate" are recorded in memory section 26. At this time, data I recorded on genome-related information recording medium 24 is accessed and whether the received "Gno." is correct or not can be confirmed.

In step C10 (SC10), in accordance with processing program 27, the morbidity rate of large-bowel cancer is displayed on display device 22 based on semantic information recorded in memory section 26. Instead of steps C8 to C10, shared computer 2 can read out (prepare) a screen that displays semantic information in accordance with processing program 13, and display it on display device 22 of personal computer 3 through communication network 1. Also in this case, semantic information is considered to be transmitted from shared computer 2 to personal computer 3. This enables the requester to obtain the morbidity rate of large-bowel cancer using genome-related information 28 recorded on genome-related information recording medium 24.

More particularly, in accordance with the flow chart shown in Fig. 19, all the genome-related information 28 recorded on genome-related information recording medium 24 is output to shared computer 2 and semantic information to provide to the requester is obtained in shared computer 2. In accordance with the flow chart shown in Fig. 19, even with a relatively small number of times of reception/transmission of information between personal computer 3 and shared computer 2, the requester can obtain semantic information. Therefore, in accordance with the flow chart shown in Fig. 19, even if the information processing capacity of personal computer 3 is relatively low, the desired semantic information can be adequately provided. In addition, the requester can obtain semantic information in a very simple manner.

As described above, according to the system, on genome-related information recording medium 24 and in main DB 14, standardization of only "polymorphism addresses" and the "polymorphism patterns" thereof eliminates the need for standardization of other specific data. Thus, the system can be utilized in a wide range of industries. That is, when providing information using genome-related information recording medium 24, the provider of objects or services can provide information in various manners without the need to standardize semantic information to correspond to the polymorphism pattern or a unified standard such as a method for transmitting/receiving data.

Furthermore, according to the system, a third party or third organization can easily monitor and control shared computer 2 by examining main DB 14. Accordingly, as the system can, for example, execute administrative control over the provider of semantic information, adequate and ethical control over the provider of semantic information can be executed.

Meanwhile, in the system for processing information, a recording medium in which information contained in data II is removed from a genome-related information recording medium, that is, a recording medium having only data I and additionally data III to V, may be used. In this case, information contained in data II is recorded in an external database (genome-related information recording medium) connected to personal computer 3 through communication network 1. In such a system, for example, in above-described step A10, the external database is accessed through communication network 1 and a polymorphism pattern in the instructed polymorphism address is read out, and the polymorphism pattern can be recorded in association with the polymorphism address in memory section 26. Thus, in this system, as with the flow charts shown in Figs. 16 and 17, 18, and 19, the requester can obtain semantic information.

In the system for processing information, the requester may have a genome-related information recording medium 24 connected to personal computer 3 through communication network 1 instead of the requester him/herself has genome-related information recording medium 24 or a recording medium in which information contained in data II is removed from the genome-related information recording medium. In such a system, the requester can access genome-related information recording medium 24 through communication network 1 to download information such as "polymorphism addresses" and "polymorphism patterns" recorded on genome-related information recording medium 24 into personal computer 3. In this case, genome-related information recording medium 24 may have genome-related information of a plurality of individuals for each individual (each "Gno.") recorded thereon.

In addition, the present invention is not limited to the above-described construction in which shared computer 2 comprises main DB 14, and, for example, is applicable to a system for processing information equipped with main DB 14 connected to shared computer 2 through communication network 1. In this case, shared computer 2 accesses main DB 14 through communication network 1 as shown in the flow charts in Figs. 16 and 17, 18 or 19. In this case also, according to the system for processing information, the requester can also obtain desired semantic information in accordance with the flow charts shown in Figs. 16 and 17, 18 or 19.

More specifically, shared computer 2 can access a plurality of main DBs 14 owned by different organizations or groups through communication network 1 and can utilize semantic information contained in these plurality of main DBs 14, thereby providing information to the requester. That is, in the system for processing information, in step A5 in the flow charts shown in Figs. 16 and 17, in step B5 in the flow chart shown in Fig. 18, or in step C5 in the flow chart shown in Fig. 19, shared computer 2 accesses various main DBs 14 having information on the morbidity rate of large-bowel cancer as semantic information. According to the system for processing information, therefore, the requester can obtain information on the morbidity rate of large-bowel cancer from information contained in various main DBs 14.

In this system, as shown in the flow charts shown in Figs. 16 and 17, 18 or 19, shared computer 2 may transmit, at least, the request information received from personal computer 3 to a so-called agent and obtain semantic information ("morbidity rate of large-bowel cancer" in this embodiment) through the agent.

All publications, patents, and patent applications cited herein are incorporated herein by reference in their entirety.

### Industrial Applicability

As is apparent from the foregoing description, the present invention can construct a system for processing information that can provide semantic information and /or information associated with the semantic information useful for an individual via effective utilization of differences in nucleotide sequence information among individuals. More particularly, the present invention enables construction of a system for processing information that can create such semantic information.

## Claims

1. A method for processing information on nucleotide sequence comprising:
(a) calculating a percentage for each piece of nucleotide sequence-related information using a first occurrence frequency and a second occurrence frequency, wherein the first occurrence frequency is calculated for each possible piece of nucleotide sequence-related information in a given position in a nucleotide sequence based on a predetermined population and the second occurrence frequency is calculated for each possible piece of nucleotide sequence-related information in the position based on the population gathered for predetermined individual-related information concerning an individual; and
(b) associating the percentage calculated in step (a) with positional information representing the position and with the nucleotide sequence-related information for each the predetermined piece of individual-related information.

2. The method for processing information according to claim 1, which further comprises step (c) of subjecting the percentage calculated in step (a) to relativization based on a predetermined standard value to obtain a relative value.

3. The method for processing information according to claim 2, which further comprises step (d) of evaluating the correlation between the predetermined individual-related information and nucleotide sequence-related information in the position by comparing the relative value obtained in step (c) with a predetermined standard value.

4. The method for processing information according to claim 3, wherein step (d) comprises determining that a larger absolute value of a difference between the relative value obtained in step (c) and the predetermined standard value indicates a stronger correlation between the predetermined individual-related information and nucleotide sequence-related information in the position.

5. The method for processing information according to claim 3, wherein step (d) comprises estimation of relevance among a plurality of pieces of the predetermined individual-related information based on a tendency regarding the positional information and the relative value of each piece of nucleotide sequence-related information concerning the predetermined individual-related information that had been determined to have the correlation.

6. The method for processing information according to claim 3, which further comprises step (e) of identifying another piece of individual-related information that had been determined to have a tendency of similar for a predetermined piece of individual-related information, which had been determined to have no correlation with nucleotide sequence-related information in step (d) and that had been determined to have correlation with nucleotide sequence-related information in step (d), and calculating an indicator for an indirect correlation between the predetermined individual-related information and nucleotide sequence-related information using a value indicating the tendency of similarity and a relative value concerning nucleotide sequence-related information that is correlated with the identified individual-related information.
